# EUROPEAN PATENT SPECIFICATION

(11) **EP 4 434 370 B1**
(45) Date of publication and mention of the grant of the patent: **12.11.2025**
(21) Application number: 23176210.5
(22) Date of filing: 30.05.2023
(51) Int. Cl.: A24F 40/51, A61M 11/04, A61M 15/06, A61M 16/10

(54) **MULTIFUNCTIONAL ATOMIZATION DEVICE**
MULTIFUNKTIONALE ZERSTÄUBUNGSVORRICHTUNG
DISPOSITIF D'ATOMISATION MULTIFONCTIONNEL

(30) Priority: 23.03.2023 CN 202310356395
(43) Date of publication of application: 25.09.2024
(73) Proprietor: Luxshare Precision Industry Company Limited, Guangdong, Shenzhen 518104 (CN)
(72) Inventor: LI, Huabing, Shenzhen, Guangdong Province 518104 (CN); LAI, Zhongyuan, Shenzhen, Guangdong Province 518104 (CN); XIAO, Chunde, Shenzhen, Guangdong Province 518104 (CN); YIN, Hongbing, Shenzhen, Guangdong Province 518104 (CN); WANG, Zijun, Shenzhen, Guangdong Province 518104 (CN); ZHOU, Xinnan, Shenzhen, Guangdong Province 518104 (CN); BAN, Honglei, Shenzhen, Guangdong Province 518104 (CN)
(74) Representative: HGF

(56) References cited:
- CN-A- 114 041 637
- US-A1- 2017 136 194
- US-A1- 2022 000 182
- US-A1- 2022 110 372
- US-A1- 2023 023 805
- US-B2- 10 799 654

## Description

### TECHNICAL FIELD

The present disclosure belongs to the technical field of atomization devices, and in particular to, a multifunctional atomization device.

### BACKGROUND

An atomization device is a device for atomizing a sample solution, and is an important component in instruments such as air humidifiers, medical atomizers or electronic cigarettes.

Currently, existing atomization devices are limited to a single function, with most atomizers only possessing the ability to atomize solutions. While a small number of atomizers on the market have environment monitoring functions, these functions are not diverse enough to accommodate a wide range of uses, resulting in a poor user experience.

Therefore, there is an urgent need for a multifunctional atomization device that solves the technical problems described above. Further relevant technologies are discussed in the patent application publications: US 2017/136194 A1 and US 2022/000182 A1. Thus, US 2017/136194 A1 relates to systems, methods, and electronic vapor devices configured to integrate advanced aromatic creation and distribution functionality via electronic vapor devices. US 2022/000182 A1 relates to a vaporizer system that includes a vaporizer device and a vaporizer accessory configured to couple to the vaporizer device.

### SUMMARY

The present disclosure provides a multifunctional atomization device as defined in the attached independent claim, and the multifunctional atomization device is used for increasing the functional diversity of the atomization device, so that the atomization device has various uses, and thus the use experience is improved. Further improvements are provided in the dependent claims.

A multifunctional atomization device includes a housing, an atomization module and an integrated module. The housing is provided with a storage cavity and an atomization cavity which are distributed along a length direction of the housing, the storage cavity is configured to store an atomized liquid, an end of the atomization cavity away from the storage cavity is provided with an air outlet communicating with an exterior of the housing, the housing is further provided with a gas channel, one end of the gas channel communicates with the exterior of the housing, and another end of the gas channel communicates with the atomization cavity. The atomization module is disposed within the atomization cavity and is configured to atomize the atomized liquid. The integrated module is disposed within the atomization cavity, and a blood oxygen detection unit, a heart rate detection unit and a gas composition detection unit are integrated on the integrated module.

In an optional technical scheme of the above-described multifunctional atomization device, the integrated module includes a block body, the block body is disposed within the atomization cavity, an end surface of an end of the block body facing the gas channel is a detection surface, and the blood oxygen detection unit, the heart rate detection unit and the gas composition detection unit are disposed on the detection surface.

In an optional technical scheme of the above-described multifunctional atomization device, a center of the detection surface is provided with a first mounting hole, multiple second mounting holes arranged in a circle whose center is the first mounting hole, and multiple third mounting holes arranged in a circle whose center is the first mounting hole are arranged on the detection surface, the multiple third mounting holes surround the multiple second mounting holes, the heart rate detection unit includes a heart rate detection member, the heart rate detection member is disposed within the first mounting hole, the blood oxygen detection unit includes multiple blood oxygen detection members, the multiple blood oxygen detection members are disposed within the multiple second mounting holes in an one-to-one correspondence, the gas composition detection unit includes multiple gas detection members, and the multiple gas detection members are disposed within the multiple third mounting holes in an one-to-one correspondence.

In the multifunctional atomization device according to the claimed invention, the atomization module includes a liquid infiltration member and a conductive heating line, the liquid infiltration member separates the atomization cavity and the storage cavity, the liquid infiltration member is configured to be infiltrated by the atomized liquid, and the conductive heating line is configured to heat the atomized liquid within the liquid infiltration member.

In the multifunctional atomization device according to the claimed invention, the conductive heating line includes a first electrode, a conductive heating structure and a second electrode, the conductive heating structure is disposed on the liquid infiltration member, one end of the first electrode is electrically connected to one end of the conductive heating structure, another end of the first electrode extends to an outer surface of the housing, one end of the second electrode is electrically connected to another end of the conductive heating structure, and another end of the second electrode extends to the outer surface of the housing.

In the multifunctional atomization device according to the claimed invention, a fixing bracket is disposed within the atomization cavity, the fixing bracket is provided with two limiting holes and a limiting groove, the limiting groove is disposed between the two limiting holes and is spaced apart from each of the two limiting holes, the integrated module is disposed within the limiting groove, and each of the first electrode and the second electrode is disposed to pass through a respective one of the two limiting holes.

In an optional technical scheme of the above-described multifunctional atomization device, a cylinder extending along the length direction is disposed within the storage cavity, one end of the cylinder is connected to an end wall of the storage cavity, another end of the cylinder is disposed to penetrate into the liquid infiltration member, and the gas channel is disposed to pass through the cylinder along the length direction.

In an optional technical scheme of the above-described multifunctional atomization device, the housing includes a main body shell and a shell cover, the shell cover covers the main body shell, one end of the liquid infiltration member is arranged within the shell cover, another end of the liquid infiltration member is arranged within the main body shell, the liquid infiltration member, an inner wall of the main body shell and an outer wall of the cylinder enclose the storage cavity, and the liquid infiltration member and an inner wall of the shell cover enclose the atomization cavity.

In a optional technical scheme of the above-described multifunctional atomization device, a first sealing ring is configured to make a seal between a circumferential outer wall of the liquid infiltration member and a circumferential inner wall of the shell cover; a second sealing ring is configured to make a seal between an inner wall of the liquid infiltration member and the outer wall of the cylinder; and a third sealing ring is configured to make a seal between the circumferential outer wall of the liquid infiltration member and a circumferential inner wall of the main body shell.

In an optional technical scheme of the above-described multifunctional atomization device, a residual liquid adsorption layer is laid on an inner end wall of an end of the atomization cavity away from the storage cavity.

The present disclosure provides a multifunctional atomization device. During use, the atomization module atomizes the atomized liquid into an atomized vapor, which is diffused within the atomization cavity. After negative pressure is generated through suction at the air outlet, external gas enters the atomization cavity through the gas channel, and the atomized vapor in the atomization cavity are dispersed through the air outlet, achieving the atomization function. Since the atomization cavity is further provided with the integrated module, the blood oxygen detection unit, the heart rate detection unit and the gas composition detection unit are integrated on the integrated module, so that the multifunctional atomization device also has the functions of measuring the blood oxygen, the heart rate and the gas composition. Therefore, the multifunctional atomization device proposed in the present disclosure offers greater versatility and usefulness, leading to an improved use experience.

### BRIEF DESCRIPTION OF DRAWINGS

In order to describe technical schemes in embodiments of the present disclosure more clearly, drawings of the embodiments of the present disclosure will be briefly described below. The drawings in the following description are merely some embodiments of the present disclosure. For those of ordinary skill in the art, other drawings may also be obtained according to the contents of the embodiments of the present disclosure and these drawings.
FIG. 1 is a structural diagram of a multifunctional atomization device according to an embodiment of the present disclosure;
FIG. 2 is a sectional diagram of a multifunctional atomization device according to an embodiment of the present disclosure;
FIG. 3 is a partial structural diagram one of a multifunctional atomization device according to an embodiment of the present disclosure;
FIG. 4 is a partial structural diagram two of a multifunctional atomization device according to an embodiment of the present disclosure;
FIG. 5 is a partial structural diagram three of a multifunctional atomization device according to an embodiment of the present disclosure;
FIG. 6 is a partial structural exploded diagram of a multifunctional atomization device according to an embodiment of the present disclosure; and
FIG. 7 is an exploded diagram of a multifunctional atomization device according to an embodiment of the present disclosure.

### Reference list

- 1: housing
- 11: main body shell
- 12: shell cover
- 121: air outlet
- 13: storage cavity
- 14: atomization cavity
- 15: gas channel
- 16: cylinder
- 2: atomization module
- 21: liquid infiltration member
- 22: conductive heating line
- 221: first electrode
- 2211: first connection post
- 2212: first contact sheet
- 222: conductive heating structure
- 223: second electrode
- 3: integrated module
- 31: block body
- 32: heart rate detection member
- 33: blood oxygen detection member
- 34: gas detection member
- 4: first sealing ring
- 5: second sealing ring
- 6: third sealing ring
- 7: fixing bracket
- 71: limiting hole
- 72: limiting groove
- 8: residual liquid adsorption layer
- 9: electricity storage battery module
- 91: electricity storage battery
- 92: first connection terminal
- 921: first sleeve portion
- 9211: first through groove
- 922: first elastic sheet
- 93: second connection terminal

### DETAILED DESCRIPTION

Embodiments of the present disclosure are described below in detail, examples of the embodiments are shown in the accompanying drawings, where the same or similar reference numerals represents the same or similar parts or parts with the same or similar functions throughout. The embodiments described below with reference to the accompanying drawings are illustrative and intended to explain the present disclosure and should not be construed as limiting the present disclosure.

In the description of the present disclosure, unless otherwise expressly specified and limited, the term "connected to each other", "connected", or "mounted" is to be construed in a broad sense, for example, as mountedly connected, or detachably connected; mechanically connected or electrically connected; directly connected to each other, indirectly connected to each other via an intermediary, internally connected between two elements, or an interaction relation between two elements. For those of ordinary skill in the art, specific meanings of the preceding terms in the present disclosure may be understood based on specific situations.

In the description of the present disclosure, unless otherwise expressly specified and limited, when a first feature is described as "above" or "below" a second feature, the first feature and the second feature may be in direct contact or be in contact via another feature between the two features. Moreover, when the first feature is described as "on", "above", or "over" the second feature, the first feature is right on, above, or over the second feature or the first feature is obliquely on, above, or over the second feature, or the first feature is simply at a higher level than the second feature. When the first feature is described as "under", "below", or "underneath" the second feature, the first feature is right under, below, or underneath the second feature or the first feature is obliquely under, below, or underneath the second feature, or the first feature is simply at a lower level than the second feature.

The technical schemes of the present disclosure are further explained by the specific implementation mode in conjunction with the attached drawings.

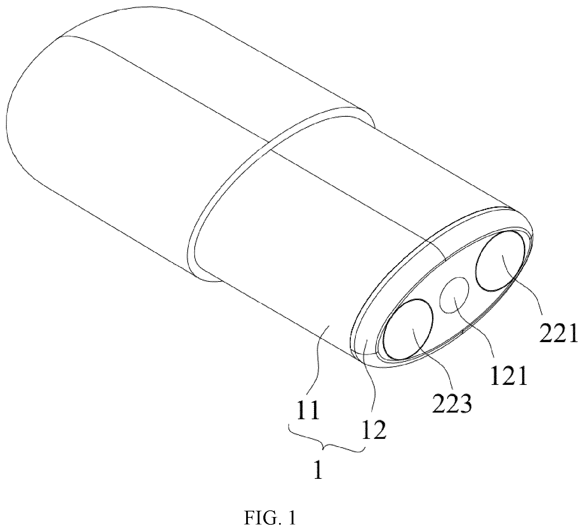

As shown in FIGS. 1 to 3, this embodiment provides a multifunctional atomization device. The multifunctional atomization device includes a housing 1, an atomization module 2, and an integrated module 3. The housing 1 is provided with a storage cavity 13 and an atomization cavity 14 which are distributed along a length direction of the housing, the storage cavity 13 is configured to store an atomized liquid, and an end of the atomization cavity 14 away from the storage cavity 13 is provided with an air outlet 121 communicating with an exterior of the housing 1. The housing 1 is further provided with a gas channel 15, one end of the gas channel 15 communicates with the exterior of the housing 1, and another end of the gas channel 15 communicates with the atomization cavity 14. The atomization module 2 is disposed within the atomization cavity 14 and is configured to atomize the atomized liquid. The integrated module 3 is disposed within the atomization cavity 14, and a blood oxygen detection unit, a heart rate detection unit and a gas composition detection unit are integrated on the integrated module 3.

According to the multifunctional atomization device provided in this embodiment, during the use, the atomization module 2 atomizes the atomized liquid into an atomized vapor, the atomized vapor is diffused within the atomization cavity 14, after a negative pressure is generated by suction at the air outlet 121, the external gas enters the atomization cavity 14 through the gas channel 15, and the atomized vapor in the atomization cavity 14 are dispersed through the air outlet 121, thereby achieving the atomization function. Since the atomization cavity 14 is further provided with the integrated module 3, the blood oxygen detection unit, the heart rate detection unit and the gas composition detection unit are integrated on the integrated module 3, so that the multifunctional atomization device also has the functions of measuring the blood oxygen, the heart rate and the gas composition. Therefore, the multifunctional atomization device proposed in this embodiment is more versatile in function and more useful in use, thereby improving the use experience of the user. Moreover, whether the harmful gas is contained in the gas is known by detecting the composition of the surrounding gas, so that the user is reminded of keeping away in time, and thus the personal safety is ensured. The physical condition of the user is obtained by detecting the blood oxygen concentration and the heart rate, so that the physical health condition may be known conveniently in time, the patient may see a doctor in due time, and thus the further aggravation of symptoms is avoided.

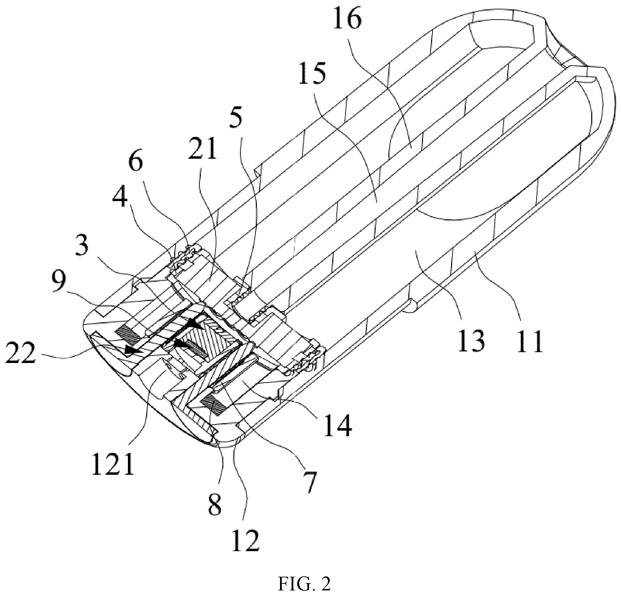

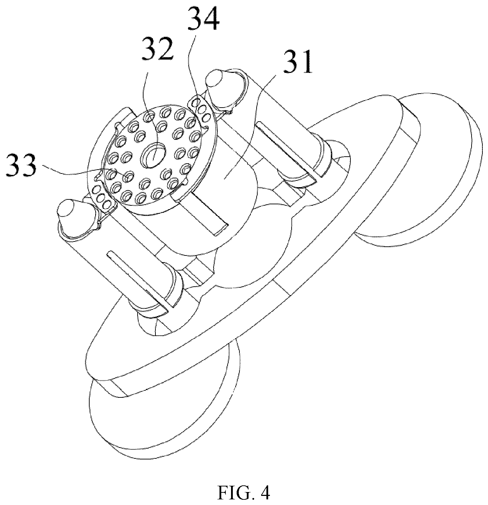

Specifically, as shown in FIGS. 2 and 4, the integrated module 3 includes a block body 31, the block body 31 is disposed within the atomization cavity 14, an end surface of an end of the block body 31 facing the gas channel 15 is a detection surface, and the blood oxygen detection unit, the heart rate detection unit and the gas composition detection unit are disposed on the detection surface. The external gas enters the atomization cavity 14 through the gas channel 15 and flows directly through the detection surface, and the gas composition detection unit detects the gas to obtain a component of the gas. The blood oxygen detection unit and the heart rate detection unit emit the light outwardly through the gas channel 15 to obtain the blood oxygen concentration and the heart rate of the user.

More specifically, a center of the detection surface is provided with a first mounting hole, multiple second mounting holes arranged in a circle whose center is the first mounting hole, and multiple third mounting holes arranged in a circle whose center is the first mounting hole are arranged on the detection surface, the multiple third mounting holes surround the multiple second mounting holes. The heart rate detection unit includes a heart rate detection member 32, the heart rate detection member 32 is disposed within the first mounting hole. The blood oxygen detection unit includes multiple blood oxygen detection members 33, the multiple blood oxygen detection members 33 are disposed within the multiple second mounting holes in an one-to-one correspondence. The gas composition detection unit includes multiple gas detection members 34, and the multiple gas detection members 34 are disposed within the multiple third mounting holes in an one-to-one correspondence. With this arrangement, the gas composition detection unit, the blood oxygen detection unit and the heart rate detection unit are integrated on the integrated module 3, so that multiple functions are simultaneously achieved, the functional diversity of the atomization device is increased, whereby the atomization device has various uses, and the use experience of the user is improved. Moreover, the detection accuracy is improved by means of distribution positions of the heart rate detection member 32, the blood oxygen detection member 33 and the gas detection member 34 on the detection surface.

Specifically, the gas detection member 34 includes a sensor for monitoring nicotine, PM2.5, formaldehyde, heavy metal, carbon monoxide and the like in the air in real time, and the gas detection member 34 detects the composition of the gas when the external gas passes through the detection surface via the gas channel 15. The specific composition and the detection principle of the gas detection member 34 may be referred to the related art, and will not be described in detail herein.

Specifically, the heart rate detection member 32 obtains the heart rate of the user by emitting the green light to the exterior of the housing 1 and further striking the green light into the skin of the user. The specific composition and the detection principle of the heart rate detection member 32 may be referred to the related art, and will not be described in detail herein.

Specifically, the blood oxygen detection member 33 obtains the blood oxygen concentration of the user by emitting the infrared ray and the red light to the exterior of the housing 1 and further striking the infrared ray and the red light into the skin of the user. The specific composition and the detection principle of the blood oxygen detection member 33 may be referred to the related art, and will not be described in detail herein.

Specifically, the integrated module 3 further includes a Bluetooth or wifi transmitting module, and the data measured by the integrated module 3 may be connected to an APP software on the mobile phone through the Bluetooth or wifi transmitting module, so that the data analysis and summarization is performed, an environment and health examination report is automatically generated, whereby the environment and the physical condition may be timely and more clearly known.

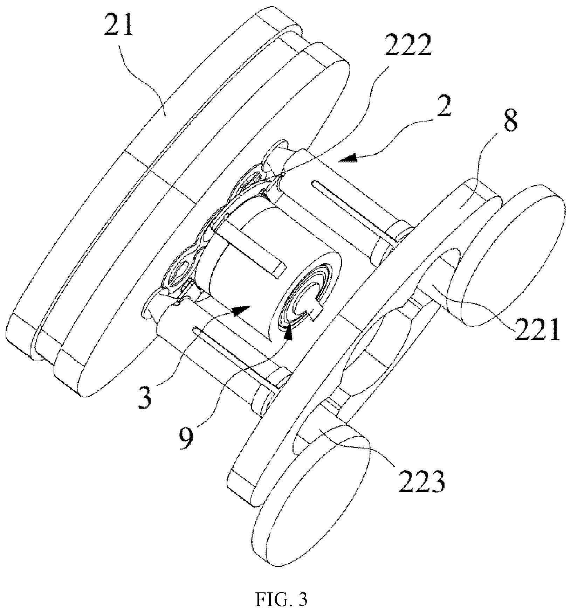

Specifically, as shown in FIGS. 2 and 3, the atomization module 2 includes a liquid infiltration member 21 and a conductive heating line 22. The liquid infiltration member 21 separates the atomization cavity 14 and the storage cavity 13, the liquid infiltration member 21 is configured to be infiltrated by the atomized liquid, and the conductive heating line 22 is configured to heat the atomized liquid within the liquid infiltration member 21. The atomized liquid is infiltrated by the liquid infiltration member 21, and the conductive heating line 22 is warmed up upon being energized, so that the atomized liquid in the atomized liquid infiltration member 21 is heated, and thus the atomization is achieved.

In this embodiment, the liquid infiltration member 21 is made of a food-grade environment-friendly high-temperature-resistant porous conforming material.

More specifically, as shown in FIGS. 1 and 3, the conductive heating line 22 includes a first electrode 221, a conductive heating structure 222 and a second electrode 223. The conductive heating structure 222 is disposed on the liquid infiltration member 21. One end of the first electrode 221 is electrically connected to one end of the conductive heating structure 222, and another end of the first electrode 221 extends to an outer surface of the housing 1. One end of the second electrode 223 is electrically connected to another end of the conductive heating structure 222, and another end of the second electrode 223 extends to the outer surface of the housing 1. Ends of the first electrode 221 and the second electrode 223 extending to the outer surface of the housing 1 interface with a power supply module. After the ends interface with the power supply module, the power supply module, the first electrode 221, the conductive heating structure 222 and the second electrode 223 form a loop, and the current is heated and warmed up upon passing through the conductive heating structure 222, thereby heating the atomized liquid. It should be appreciated that the power supply module is a structure which is used for being matched and connected with the multifunctional atomization device and supplying power to the multifunctional atomization device.

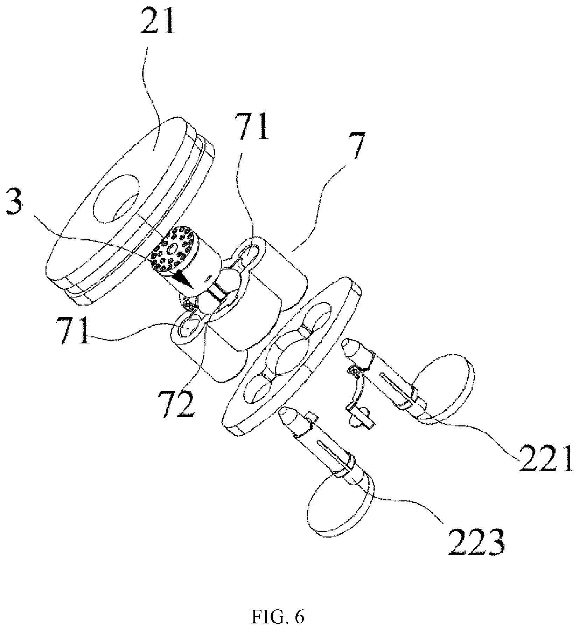

Specifically, as shown in FIGS. 2 and 6, a fixing bracket 7 is disposed within the atomization cavity 14, the fixing bracket 7 is provided with two limiting holes 71 and a limiting groove 72, the limiting groove 72 is disposed between the two limiting holes 71 and is spaced apart from each of the two limiting holes 71, the integrated module 3 is disposed within the limiting groove 72, and each of the first electrode 221 and the second electrode 223 is disposed to pass through a respective one of the two limiting holes 71. The positioning of the integrated module 3 and the first electrode 221 and the second electrode 223 is achieved by the fixing bracket 7.

Specifically, a cylinder 16 extending along the length direction is disposed within the storage cavity 13, one end of the cylinder 16 is connected to an end wall of the storage cavity 13, another end of the cylinder 16 is disposed to penetrate into the liquid infiltration member 21, and the gas channel 15 is disposed to pass through the cylinder 16 along the length direction. The external gas may enter the atomization cavity 14 through the gas channel 15 so as to balance the pressure inside and outside the atomization cavity 14, thereby ensuring that the atomized vapor formed by atomization within the atomization cavity 14 may be dispersed through the air outlet 121.

Specifically, as shown in FIGS. 1 and 2, the housing 1 includes a main body shell 11 and a shell cover 12, the shell cover 12 covers the main body shell 11, one end of the liquid infiltration member 21 is arranged within the shell cover 12, and another end of the liquid infiltration member 21 is arranged within the main body shell 11. The liquid infiltration member 21, an inner wall of the main body shell 11, and an outer wall of the cylinder 16 enclose the storage cavity 13, and the liquid infiltration member 21 and the inner wall of the shell cover 12 enclose the atomization cavity 14. The liquid infiltration member 21 separates the storage cavity 13 and the atomization cavity 14, so that the atomized liquid in the storage cavity 13 is prevented from flowing into the atomization cavity 14, and the heating atomization of the atomized liquid by the conductive heating line 22 can be achieved. The main body shell 11 and the shell cover 12 are connected in a covering manner to facilitate the placement of the components and the atomized liquid in the housing 1.

In this embodiment, both the main body shell 11 and the shell cover 12 are made of a food environment-friendly-grade material.

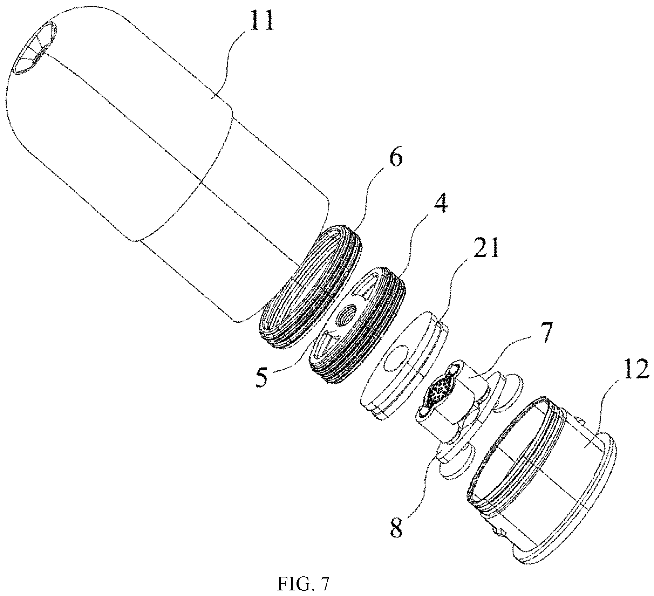

Specifically, as shown in FIGS. 2 and 7, a first sealing ring 4 is configured to make a seal between a circumferential outer wall of the liquid infiltration member 21 and a circumferential inner wall of the shell cover 12, and the first sealing ring 4 improves the sealing of the connection between the liquid infiltration member 21 and the shell cover 12. A second sealing ring 5 is configured to make a seal between an inner wall of the liquid infiltration member 21 and the outer wall of the cylinder 16, and the second sealing ring 5 improves the sealing of the connection between the liquid infiltration member 21 and the cylinder 16. A third sealing ring 6 is configured to make a seal between the circumferential outer wall of the liquid infiltration member 2 and a circumferential inner wall of the main body shell 11, and the third sealing ring 6 improves the sealing of the connection between the liquid infiltration member 21 and the main body shell 11. With this arrangement, the sealing between the storage cavity 13 and the atomization cavity 14 and the sealing at the junction of the main body shell 11 and the shell cover 12 are ensured, the atomized liquid within the storage cavity 13 is prevented from flowing into the atomization cavity 14, the normal operation of the components in the atomization cavity 14 is ensured, and the atomized liquid within the storage cavity 13 is prevented from leaking out through the junction of the main body shell 11 and the shell cover 12.

Specifically, as shown in FIG. 7, the first sealing ring 4 and the second sealing ring 5 are connected and integrally formed, whereby the manufacturing efficiency is improved, and the sealing effect is improved.

More specifically, inner and outer surfaces of the first sealing ring 4, inner and outer surfaces of the second sealing ring 5, and inner and outer surfaces of the third sealing ring 6 are corrugated surfaces, thereby further improving the sealing.

In this embodiment, the first sealing ring 4, the second sealing ring 5 and the third sealing ring 6 are all made of food-grade environment-friendly silica gel.

Specifically, as shown in FIG. 2, the third sealing ring 6 is configured to make a seal between the first sealing ring 4 and the circumferential inner wall of the main body shell 11.

Specifically, with continued reference to FIG. 2, a residual liquid adsorption layer 8 is laid on an inner end wall of an end of the atomization cavity 14 away from the storage cavity 13. The atomized vapor formed by atomizing the atomization module 2 is dispersed within the atomization cavity 14, when the air pressure at the air outlet 121 decreases, the atomized vapor in the atomization cavity 14 is discharged through the air outlet 121 due to the action of the air pressure difference. During the use, a small amount of atomized vapor will liquefy within the atomization cavity 14, and the residual liquid adsorption layer 8 is used for adsorbing these liquefied liquids so as not to avoid the liquid form affecting the normal operation of the components.

In this embodiment, the residual liquid adsorption layer 8 is made of a food-grade environment-friendly condensed fiber.

Specifically, as shown in FIG. 2, the multifunctional atomization device provided in this embodiment further includes an electricity storage battery module 9, the electricity storage battery module 9 is disposed within the atomization cavity 14, the electricity storage battery module 9 is electrically connected to the integrated module 3 and may supply power to the integrated module 3, the electricity storage battery module 9 is electrically connected to the conductive heating line 22, and the electricity storage battery module 9 is charged when the conductive heating line 22 is energized.

According to the multifunctional atomization device provided in this embodiment, when the atomizing is to be performed, the conductive heating line 22 is energized, and the conductive heating line 22 heats the atomized liquid to achieve the atomization, and meanwhile, the electricity storage battery module 9 is charged, after the conductive heating line 22 is de-energized, the atomization ends. Since the electricity is stored in the electricity storage battery module 9, the integrated module 3 may be separately supplied with electricity, that is, the multifunctional atomization device may detect the surrounding gas independently without the atomization. Therefore, the integrated module 3 may independently work, so that the use method of the multifunctional atomization device is flexible and diversified, and the user experience is improved.

Specifically, a positive electrode of the electricity storage battery module 9 is electrically connected to the first electrode 221, and a negative electrode of the electricity storage battery module 9 is electrically connected to the second electrode 223. After the ends of the first electrode 221 and the second electrode 223 extending to the outer surface of the housing 1 interface with the power supply module, the power supply module, the first electrode 221, the conductive heating structure 222 and the second electrode 223 form a loop, and the current is heated and warmed up upon passing through the conductive heating structure 222, thereby heating the atomized liquid. At the same time of the atomization, the current flows through the electricity storage battery module 9 through the first electrode 221 and the second electrode 223, and the electricity storage battery module 9 is configured to store the electric energy.

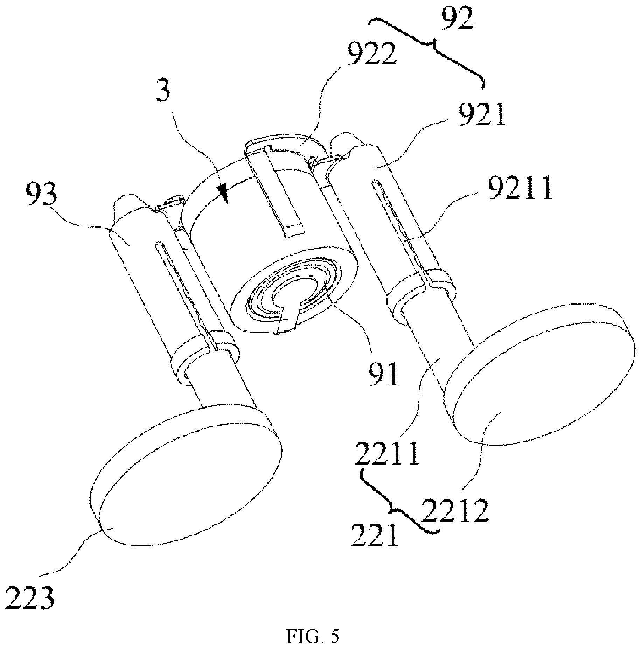

Specifically, as shown in FIG. 5, the electricity storage battery module 9 includes an electricity storage battery 91, a first connection terminal 92 and a second connection terminal 93, the electricity storage battery 91 is electrically connected to the integrated module 3, one end of the first connection terminal 92 is electrically connected to the first electrode 221, another end of first connection terminal 92 is electrically connected to a positive electrode of the electricity storage battery 91, one end of the second connection terminal 93 is electrically connected to the second electrode 223, and another end of the second connection terminal 93 is electrically connected to a negative electrode of the electricity storage battery 91. The first connection terminal 92 achieves the electrical connection between the first electrode 221 and the positive electrode of the electricity storage battery 91, the second connection terminal 93 achieves the electrical connection between the second electrode 223 and the negative electrode of the electricity storage battery 91, and the electricity storage battery 91 is configured to store the electric energy.

More specifically, the first connection terminal 92 includes a first sleeve portion 921 and a first elastic sheet 922, the first sleeve portion 921 is sleeved outside the first electrode 221, one end of the first elastic sheet 922 is connected to the first sleeve portion 921, and another end of the first elastic sheet 922 is fused with the positive electrode of the electricity storage battery 91. The second connection terminal 93 includes a second sleeve portion and a second elastic sheet, the second sleeve portion is sleeved outside the second electrode 223, one end of the second elastic sheet is connected to the second sleeve portion, and another end of the second elastic sheet is fused with the negative electrode of the current electricity storage battery 91. The first sleeve portion 921 and the first electrode 221 are sleeved, so that the firmness of the electrical connection between the first sleeve portion 921 and the first electrode 221 is improved. The first elastic sheet 922 and the electricity storage battery 91 are fused, so that the firmness of the electrical connection between the first elastic sheet 922 and the electricity storage battery 91 is improved. Similarly, the second sleeve portion and the second electrode 223 are sleeved, so that the firmness of the electrical connection between the second sleeve portion and the second electrode 223 is improved, the second elastic sheet and the electricity storage battery 91 are fused, so that the firmness of the electrical connection between the second elastic sheet and the electricity storage battery 91. The first elastic sheet 922 and the second elastic sheet 922 have the resilient cushioning effect, so that the electrical connections are prevented from being disconnected due to shaking, thereby further ensuring the firmness of the electrical connection.

In this embodiment, the first elastic sheet 922 is fused with the electricity storage battery 91 by means of laser or infrared light. The second elastic sheet is fused with the electricity storage battery 91 by means of laser or infrared light.

Further specifically, an outer side wall of the first sleeve portion 921 is provided with multiple first through grooves 9211 communicating with the interior of the first sleeve portion 921, one end of the first through groove 9211 extends to an end of one end of the first sleeve portion 921, and the first elastic sheet 922 is connected to another end of the first sleeve portion 921. An outer side wall of the second sleeve portion is provided with multiple second through grooves communicating with the interior of the second sleeve portion, one end of the second through groove extends to an end of one end of the second sleeve portion, and the second elastic sheet is connected to another end of the second sleeve portion. The first through groove 9211 is provided so that a side wall of the first sleeve portion 921 may be elastically deformed to some extent, and thus, the first electrode 221 may be clamped. Similarly, the second through groove is provided so that a side wall of the second sleeve portion may be elastically deformed to some extent, and thus, the second electrode 223 may be clamped.

Specifically, the integrated module 3 is disposed between the first electrode 221 and the second electrode 223, and the electricity storage battery 91 is disposed within a mounting groove at one end of the integrated module 3. With this arrangement, the electrical connection line between the integrated module 3 and the electricity storage battery 91 is relatively short, moreover, the distance between the first electrode 221 and the second electrode 223 and the electricity storage battery 91 is relatively small, so that the lengths of the first connection terminal 92 and the second connection terminal 93 are relatively small, which is advantageous for reducing the cost of the material. Furthermore, the structure is compact and the occupied space is small, thereby avoiding increasing the volume of the multifunctional atomization device due to the increase of the electricity storage battery module 9.

In this embodiment, an end face of one end of the block body 31 of the integrated module 3 away from the detection surface is provided with a mounting groove for accommodating the electricity storage battery 91. The electricity storage battery 91 is disposed within the limit groove 72 in a manner that one end of the mounting groove faces the groove bottom of the limiting groove 72 on the fixing bracket 7, so that the electricity storage battery 91 is fixed firmly.

Specifically, as shown in FIGS. 1, 3, and 5, the first electrode 221 includes a first connection post 2211 and a first contact sheet 2212 connected to one end of the first connection post 2211, an end of the first connection post 2211 away from the first contact sheet 2212 is electrically connected to the conductive heating structure 222, the first contact sheet 2212 is embedded on the outer surface of the housing 1, and the first connection post 2211 is electrically connected to the positive electrode of the electricity storage battery module 9. The second electrode 223 includes a second connection post and a second contact sheet connected to one end of the second connection post, an end of the second connection post away from the second contact sheet is electrically connected to the conductive heating structure 222, the second contact sheet is embedded on the outer surface of the housing 1, and the second connection post is electrically connected to the negative electrode of the electricity storage battery module 9. The first contact sheet 2212 and the second contact sheet increase the contact area with the power supply module, thereby facilitating the power supply of the power supply module to the multifunctional atomization device.

With continued reference to FIGS. 1, 3, and 5, in this embodiment, the first sleeve portion 921 is sleeved outside the first connection post 2211, one end provided with the first through groove 9211 of the first sleeve portion 921 is disposed close to the first contact sheet 2212, and one end of the first connection post 2211 away from the first contact sheet 2212 is provided with a conical head, and the conical head facilitates penetration into the first sleeve portion 921 to facilitate the assembly of the first electrode 221 and the first connection terminal 92. Similarly, the second sleeve portion is sleeved outside the second connection post, one end provided with the second through groove of the second sleeve portion is disposed close to the second contact sheet, and an end of the second connection post away from the second contact sheet is provided with a conical head, the conical head facilitates penetration into the second sleeve portion to facilitate the assembly of the second electrode 223 and the second connection terminal 93.

## Claims

1. A multifunctional atomization device, comprising:
a housing (1) provided with a storage cavity (13) and an atomization cavity (14) which are distributed along a length direction of the housing (1), wherein the storage cavity (13) is configured to store an atomized liquid, an end of the atomization cavity (14) away from the storage cavity (13) is provided with an air outlet (121) communicating with an exterior of the housing (1), the housing (1) is further provided with a gas channel (15), one end of the gas channel (15) communicates with the exterior of the housing (1), and another end of the gas channel (15) communicates with the atomization cavity (14);
an atomization module (2) being disposed within the atomization cavity (14) and being configured to atomize the atomized liquid; and
an integrated module (3) disposed within the atomization cavity (14), wherein a blood oxygen detection unit, a heart rate detection unit and a gas composition detection unit are integrated on the integrated module (3);
wherein the atomization module (2) comprises a liquid infiltration member (21) and a conductive heating line (22), the liquid infiltration member (21) separates the atomization cavity (14) and the storage cavity (13), the liquid infiltration member (21) is configured to be infiltrated by the atomized liquid, and the conductive heating line (22) is configured to heat the atomized liquid within the liquid infiltration member (21);
wherein the conductive heating line (22) comprises a first electrode (221), a conductive heating structure (222) and a second electrode (223), the conductive heating structure (222) is disposed on the liquid infiltration member (21), one end of the first electrode (221) is electrically connected to one end of the conductive heating structure (222), another end of the first electrode (221) extends to an outer surface of the housing (1), one end of the second electrode (223) is electrically connected to another end of the conductive heating structure (222), and another end of the second electrode (223) extends to the outer surface of the housing (1);
the multifunctional atomization device is **characterized in that** a fixing bracket (7) is disposed within the atomization cavity (14), the fixing bracket (7) is provided with two limiting holes (71) and a limiting groove (72), the limiting groove (72) is disposed between the two limiting holes (71) and is spaced apart from each of the two limiting holes (71), the integrated module (3) is disposed within the limiting groove (72), and each of the first electrode (221) and the second electrode (223) is disposed to pass through a respective one of the two limiting holes (71).

2. The multifunctional atomization device of claim 1, wherein the integrated module (3) comprises a block body (31), the block body (31) is disposed within the atomization cavity (14), an end surface of an end of the block body (31) facing the gas channel (15) is a detection surface, and the blood oxygen detection unit, the heart rate detection unit and the gas composition detection unit are disposed on the detection surface.

3. The multifunctional atomization device of claim 2, wherein a center of the detection surface is provided with a first mounting hole, a plurality of second mounting holes arranged in a circle whose center is the first mounting hole, and a plurality of third mounting holes arranged in a circle whose center is the first mounting hole are arranged on the detection surface, the plurality of third mounting holes surround the plurality of second mounting holes, the heart rate detection unit comprises a heart rate detection member (32), the heart rate detection member (32) is disposed within the first mounting hole, the blood oxygen detection unit comprises a plurality of blood oxygen detection members (33), the plurality of blood oxygen detection members (33) are disposed within the plurality of second mounting holes in an one-to-one correspondence, the gas composition detection unit comprises a plurality of gas detection members (34), and the plurality of gas detection members (34) are disposed within the plurality of third mounting holes in an one-to-one correspondence.

4. The multifunctional atomization device of claim 1, wherein a cylinder (16) extending along the length direction is disposed within the storage cavity (13), one end of the cylinder (16) is connected to an end wall of the storage cavity (13), another end of the cylinder (16) is disposed to penetrate into the liquid infiltration member (21), and the gas channel (15) is disposed to pass through the cylinder (16) along the length direction.

5. The multifunctional atomization device of claim 4, wherein the housing (1) comprises a main body shell (11) and a shell cover (12), the shell cover (12) covers the main body shell (11), one end of the liquid infiltration member (21) is arranged within the shell cover (12), another end of the liquid infiltration member (21) is arranged within the main body shell (11), the liquid infiltration member (21), an inner wall of the main body shell (11) and an outer wall of the cylinder (16) enclose the storage cavity (13), and the liquid infiltration member (21) and an inner wall of the shell cover (12) enclose the atomization cavity (14).

6. The multifunctional atomization device of claim 5, wherein a first sealing ring (4) is configured to make a seal between a circumferential outer wall of the liquid infiltration member (21) and a circumferential inner wall of the shell cover (12);
a second sealing ring (5) is configured to make a seal between an inner wall of the liquid infiltration member (21) and the outer wall of the cylinder (16); and
a third sealing ring (6) is configured to make a seal between the circumferential outer wall of the liquid infiltration member (21) and a circumferential inner wall of the main body shell (11).

7. The multifunctional atomization device of any one of claims 1 to 6, wherein a residual liquid adsorption layer (8) is laid on an inner end wall of an end of the atomization cavity (14) away from the storage cavity (13).

## Patentansprüche

1. Multifunktionale Zerstäubungsvorrichtung, umfassend:
ein Gehäuse (1), das mit einem Speicherhohlraum (13) und einem Zerstäubungshohlraum (14) versehen ist, die entlang einer Längsrichtung des Gehäuses (1) verteilt sind, wobei der Speicherhohlraum (13) zum Speichern einer Zerstäubungsflüssigkeit konfiguriert ist, wobei ein dem Speicherhohlraum (13) abgewandtes Ende des Zerstäubungshohlraums (14) mit einem Luftauslass (121) versehen ist, der mit einem Exterieur des Gehäuses (1) in Verbindung steht, wobei das Gehäuse (1) ferner mit einem Gaskanal (15) versehen ist, wobei ein Ende des Gaskanals (15) mit dem Exterieur des Gehäuses (1) in Verbindung steht und ein anderes Ende des Gaskanals (15) mit dem Zerstäubungshohlraum (14) in Verbindung steht;
ein Zerstäubungsmodul (2), das innerhalb des Zerstäubungshohlraums (14) angeordnet und so konfiguriert ist, dass es die Zerstäubungsflüssigkeit zerstäubt; und
ein Integralmodul (3), das innerhalb des Zerstäubungshohlraums (14) angeordnet ist, wobei eine Blutsauerstoff-Erfassungseinheit, eine Herzfrequenz-Erfassungseinheit und eine Gaszusammensetzungs-Erfassungseinheit an dem Integralmodul (3) integriert sind;
wobei das Zerstäubungsmodul (2) ein Flüssigkeitsinfiltrationselement (21) und eine leitfähige Heizleitung (22) umfasst, wobei das Flüssigkeitsinfiltrationselement (21) den Zerstäubungshohlraum (14) und den Speicherhohlraum (13) trennt, wobei das Flüssigkeitsinfiltrationselement (21) so konfiguriert ist, dass es von der Zerstäubungsflüssigkeit infiltriert wird, und wobei die leitfähige Heizleitung (22) so konfiguriert ist, dass sie die Zerstäubungsflüssigkeit innerhalb des Flüssigkeitsinfiltrationselements (21) erhitzt;
wobei die leitfähige Heizleitung (22) eine erste Elektrode (221), eine leitfähige Heizstruktur (222) und eine zweite Elektrode (223) umfasst, wobei die leitfähige Heizstruktur (222) an dem Flüssigkeitsinfiltrationselement (21) angeordnet ist, wobei ein Ende der ersten Elektrode (221) elektrisch mit einem Ende der leitfähigen Heizstruktur (222) verbunden ist und sich ein anderes Ende der ersten Elektrode (221) zu einer Außenfläche des Gehäuses (1) erstreckt, wobei ein Ende der zweiten Elektrode (223) elektrisch mit einem anderen Ende der leitfähigen Heizstruktur (222) verbunden ist und sich ein anderes Ende der zweiten Elektrode (223) zur Außenfläche des Gehäuses (1) erstreckt;
wobei die multifunktionale Zerstäubungsvorrichtung **dadurch gekennzeichnet ist, dass** innerhalb des Zerstäubungshohlraums (14) eine Befestigungshalterung (7) angeordnet ist, die mit zwei Begrenzungslöchern (71) und einer Begrenzungsnut (72) versehen ist, wobei die Begrenzungsnut (72) zwischen den beiden Begrenzungslöchern (71) angeordnet ist und von jedem der beiden Begrenzungslöcher (71) beabstandet ist, wobei das Integralmodul (3) innerhalb der Begrenzungsnut (72) angeordnet ist, und wobei die erste Elektrode (221) und die zweite Elektrode (223) jeweils so angeordnet sind, dass sie durch ein jeweiliges der beiden Begrenzungslöcher (71) hindurchgehen.

2. Multifunktionale Zerstäubungsvorrichtung nach Anspruch 1, wobei das Integralmodul (3) einen Blockkörper (31) umfasst, der innerhalb des Zerstäubungshohlraums (14) angeordnet ist, wobei eine Stirnfläche eines dem Gaskanal (15) zugewandten Endes des Blockkörpers (31) eine Erfassungsfläche ist, und wobei die Blutsauerstoff-Erfassungseinheit, die Herzfrequenz-Erfassungseinheit und die Gaszusammensetzungs-Erfassungseinheit an der Erfassungsfläche angeordnet sind.

3. Multifunktionale Zerstäubungsvorrichtung nach Anspruch 2, wobei eine Mitte der Erfassungsfläche mit einem ersten Montageloch versehen ist, wobei eine Vielzahl von zweiten Montagelöchern, die in einem Kreis angeordnet sind, dessen Mittelpunkt das erste Montageloch ist, und eine Vielzahl von dritten Montagelöchern, die in einem Kreis angeordnet sind, dessen Mittelpunkt das erste Montageloch ist, an der Erfassungsfläche angeordnet sind, wobei die Vielzahl von dritten Montagelöchern die Vielzahl von zweiten Montagelöchern umgeben, wobei die Herzfrequenz-Erfassungseinheit ein Herzfrequenz-Erfassungselement (32) umfasst, das innerhalb des ersten Montagelochs angeordnet ist, wobei die Blutsauerstoff-Erfassungseinheit eine Vielzahl von Blutsauerstoff-Erfassungselementen (33) umfasst, die eineindeutig innerhalb der Vielzahl von zweiten Montagelöchern angeordnet sind, wobei die Gaszusammensetzungs-Erfassungseinheit eine Vielzahl von Gas-Erfassungselementen (34) umfasst, die eineindeutig innerhalb der Vielzahl von dritten Montagelöchern angeordnet sind.

4. Multifunktionale Zerstäubungsvorrichtung nach Anspruch 1, wobei ein sich in Längsrichtung erstreckender Zylinder (16) innerhalb des Speicherhohlraums (13) angeordnet ist, wobei ein Ende des Zylinders (16) mit einer Stirnwand des Speicherhohlraums (13) verbunden ist, wobei ein anderes Ende des Zylinders (16) so angeordnet ist, dass es in das Flüssigkeitsinfiltrationselement (21) hineinragt, und wobei der Gaskanal (15) so angeordnet ist, dass er durch den Zylinder (16) entlang der Längsrichtung hindurchgeht.

5. Multifunktionale Zerstäubungsvorrichtung nach Anspruch 4, wobei das Gehäuse (1) eine Hauptkörperhülle (11) und eine Hüllenabdeckung (12) umfasst, die die Hauptkörperhülle (11) abdeckt, wobei ein Ende des Flüssigkeitsinfiltrationselements (21) innerhalb der Hüllenabdeckung (12) angeordnet ist, wobei ein anderes Ende des Flüssigkeitsinfiltrationselements (21) innerhalb der Hauptkörperhülle (11) angeordnet ist, wobei das Flüssigkeitsinfiltrationselement (21), eine Innenwand der Hauptkörperhülle (11) und eine Außenwand des Zylinders (16) den Speicherhohlraum (13) umschließen, und wobei das Flüssigkeitsinfiltrationselement (21) und eine Innenwand der Hüllenabdeckung (12) den Zerstäubungshohlraum (14) umschließen.

6. Multifunktionale Zerstäubungsvorrichtung nach Anspruch 5, wobei ein erster Dichtungsring (4) so konfiguriert ist, dass er eine Dichtung zwischen einer umlaufenden Außenwand des Flüssigkeitsinfiltrationselements (21) und einer umlaufenden Innenwand der Hüllenabdeckung (12) bildet;
wobei ein zweiter Dichtungsring (5) so konfiguriert ist, dass er eine Dichtung zwischen einer Innenwand des Flüssigkeitsinfiltrationselements (21) und der Außenwand des Zylinders (16) bildet; und
wobei ein dritter Dichtungsring (6) so konfiguriert ist, dass er eine Dichtung zwischen der umlaufenden Außenwand des Flüssigkeitsinfiltrationselements (21) und einer umlaufenden Innenwand der Hauptkörperhülle (11) bildet.

7. Multifunktionale Zerstäubungsvorrichtung nach einem der Ansprüche 1 bis 6, wobei eine Restflüssigkeits-Adsorptionsschicht (8) an einer inneren Stirnwand eines dem Speicherhohlraum (13) abgewandten Endes des Zerstäubungshohlraums (14) angeordnet ist.

## Revendications

1. Dispositif d'atomisation multifonctionnel, comprenant :
un logement (1) muni d'une cavité de stockage (13) et une cavité d'atomisation (14) qui sont réparties dans le sens de la longueur du logement (1), dans lequel la cavité de stockage (13) est configurée pour stocker un liquide atomisé, une terminaison de la cavité d'atomisation (14) distale de la cavité de stockage (13) est munie d'une sortie d'air (121) communicant avec l'extérieur du logement (1), le logement (1) est en outre uni d'un canal de gaz (15), une terminaison du canal de gaz (15) communique avec l'extérieur du logement (1), et une autre terminaison du canal de gaz (15) communique avec la cavité d'atomisation (14) ;
un module d'atomisation (2) étant agencé dans la cavité d'atomisation (14) et étant configuré pour atomiser le liquide atomisé ; et
un module intégré (3) agencé dans la cavité d'atomisation (14), dans lequel une unité de détection d'oxygène sanguin, une unité de détection de fréquence cardiaque et une unité de détection de composition de gaz sont intégrés dans le module intégré (3) ;
dans lequel le module d'atomisation (2) comprend un élément d'infiltration de liquide (21) et une ligne de chauffage conductrice (22), l'élément d'infiltration de liquide (21) sépare la cavité d'atomisation (14) et la cavité de stockage (13), l'élément d'infiltration de liquide (21) est configuré pour être infiltré par le liquide atomisé, et la ligne de chauffage conductrice (22) est configurée pour chauffer le liquide atomisé dans l'élément d'infiltration de liquide (21) ;
dans lequel la ligne de chauffage conductrice (22) comprend une première électrode (221), une structure de chauffage conductrice (222) et une deuxième électrode (223), la structure de chauffage conductrice (222) est agencée sur l'élément d'infiltration de liquide (21), une terminaison de la première électrode (221) est raccordée électriquement à une terminaison de la structure de chauffage conductrice (222), une autre terminaison de la première électrode (221) se prolonge jusqu'à une surface externe du logement (1), une terminaison de la deuxième électrode (223) est raccordée électriquement à une autre terminaison de la structure de chauffage conductrice (222), et une autre terminaison de la deuxième électrode (223) se prolonge jusqu'à la surface externe du logement (1) ;
le dispositif d'atomisation multifonctionnel est **caractérisé en ce qu'**un support de fixation (7) est agencé à l'intérieur de la cavité d'atomisation (14), le support de fixation (7) est muni de deux trous limitants (71) et d'une gorge limitante (72), la gorge limitante (72) est agencée entre les deux trous limitants (71) et est espacée de chacun des deux trous limitants (71), le module intégré (3) est agencé à l'intérieur de la gorge limitante (72), et chacune de la première électrode (221) et de la deuxième électrode (223) est agencée pour passer à travers l'un des deux trous limitants (71) respectif.

2. Dispositif d'atomisation multifonctionnel selon la revendication 1, dans lequel le module intégré (3) comprend un corps de bloc (31), le corps de bloc (31) est agencé à l'intérieur de la cavité d'atomisation (14), une surface terminale d'une terminaison du corps de bloc (31) faisant face au canal de gaz (15) est une surface détection, et l'unité de détection d'oxygène sanguin, l'unité de détection de fréquence cardiaque et l'unité de détection de composition de gaz sont agencées sur la surface de détection.

3. Dispositif d'atomisation multifonctionnel selon la revendication 2, dans lequel le centre de la surface de détection est muni d'un premier trou de montage, une pluralité de deuxièmes trous de montage agencés en un cercle dont le centre est le premier trou de montage, et une pluralité de troisièmes trous de montage agencés en un cercle dont le centre est le premier trou de montage sont agencés sur la surface de détection, la pluralité de troisièmes trous de montage entoure la pluralité de deuxièmes trous de montage, l'unité de détection de fréquence cardiaque comprend un élément de détection de fréquence cardiaque (32), l'élément de détection de fréquence cardiaque (32) est agencé à l'intérieur du premier trou de montage, l'unité de détection d'oxygène sanguin comprend une pluralité d'éléments de détection d'oxygène sanguin (33), la pluralité d'éléments de détection d'oxygène sanguin (33) est agencée à l'intérieur de la pluralité de deuxièmes trous de montage en correspondance un-à-un, l'unité de détection de composition de gaz comprend une pluralité d'éléments de détection de gaz (34), et la pluralité d'éléments de détection de gaz (34) est agencée à l'intérieur de la pluralité de troisièmes trous de montage en correspondance un-à-un.

4. Dispositif d'atomisation multifonctionnel selon la revendication 1, dans lequel un cylindre (16) se prolongeant dans le sens de la longueur est agencé à l'intérieur de la cavité de stockage (13), une terminaison du cylindre (16) est raccordée à une paroi terminale de la cavité de stockage (13), une autre terminaison du cylindre (16) est agencée pour pénétrer dans l'élément d'infiltration de liquide (21), et le canal de gaz (15) est agencé pour passer à travers le cylindre (16) dans le sens de la longueur.

5. Dispositif d'atomisation multifonctionnel selon la revendication 4, dans lequel le logement (1) comprend une coque de corps principal (11) et un couvercle de coque (12), le couvercle de coque (12) recouvre la coque de corps principal (11), une terminaison de l'élément d'infiltration de liquide (21) est agencée à l'intérieur du couvercle de coque (12), une autre terminaison de l'élément d'infiltration de liquide (21) est agencée à l'intérieur de la coque de corps principal (11), l'élément d'infiltration de liquide (21), une paroi interne de la coque de corps principal (11) et une paroi externe du cylindre (16) enferment la cavité de stockage (13), et l'élément d'infiltration de liquide (21) et une paroi interne du couvercle de coque (12) enferment la cavité d'atomisation (14).

6. Dispositif d'atomisation multifonctionnel selon la revendication 5, dans lequel une première bague d'étanchéité (4) est configurée pour faire un scellement entre une paroi externe circonférentielle de l'élément d'infiltration de liquide (21) et une paroi interne circonférentielle du couvercle de coque (12) ;
une deuxième bague d'étanchéité (5) est configurée pour faire un scellement entre une paroi interne de l'élément d'infiltration de liquide (21) et la paroi externe du cylindre (16) ; et
une troisième bague d'étanchéité (6) est configuré pour faire un scellement entre la paroi externe circonférentielle de l'élément d'infiltration de liquide (21) et une paroi interne circonférentielle de la coque de corps principal (11).

7. Dispositif d'atomisation multifonctionnel selon l'une quelconque des revendications 1 à 6, dans lequel une couche d'adsorption de liquide résiduel (8) est posée sur une paroi terminale interne d'une terminaison de la cavité d'atomisation (14) distale de la cavité de stockage (13).
